# EUROPEAN PATENT SPECIFICATION

(11) **EP 1 531 922 B1**
(45) Date of publication and mention of the grant of the patent: **29.06.2011**
(21) Application number: 03792376.0
(22) Date of filing: 19.08.2003
(51) Int. Cl.: B01D 39/16, A61M 1/36

(54) **FILTER FOR THE DEPLETION OF LEUKOCYTES FROM BLOOD PRODUCTS**
FILTER ZUR ENTFERNUNG VON LEUKOZYTEN AUS BLUTPRODUKTEN
FILTRE DESTINE A REALISER LA DEPLETION DES LEUCOCYTES DE PRODUITS SANGUINS

(30) Priority: 21.08.2002 IT TO20020736
(43) Date of publication of application: 25.05.2005
(73) Proprietor: Fresenius Hemocare Italia S.r.l., I-41032 Cavezzo, (Modena) (IT)
(72) Inventor: MARI, Giorgio, I-41037 Mirandola (IT); VERRI, Paolo, I-41037 Mirandola (IT); ORI, Alessandra, I-41100 Modena (IT)
(74) Representative: Richly, Erik
(86) International application number: PCT/EP2003/009174
(87) International publication number: WO 2004/018078

(56) References cited:
- EP-A- 0 313 348
- EP-A- 0 542 655
- EP-A- 1 093 823
- WO-A-00/20053

## Description

The present invention relates to a filter device for the depletion of the leukocyte content from blood products such as whole blood and/or blood components.

More particularly, the invention relates to a leukocyte filter device adapted for use in the blood bag systems which are conventionally used for the separation of whole blood into leukocyte depleted hemocomponents.

Blood bag systems are known e.g. from US-4,596,657, EP-A-0 556 303 and EP-A-0 879 608.

US-4,596,657 describes a blood bag system including a primary bag which is connected by means of flexible hose lines to a first and a second satellite bag; a filtering means is integrally disposed between the primary bag and one satellite bag to remove platelets and leukocytes from a mixture of a red cell concentrate and additive solution which is passed through the filtering means from the primary bag to the said satellite bag.

EP-A-0 556 303 and US-5,100,564 describe a blood collection and processing system for preparing, from donated whole blood, platelet-rich plasma (PRP), packed red cells (PRC), platelet concentrate (PC) and plasma; in the described system, PRP is leukocyte depleted by interposing in the conduit between a blood collection bag and a first satellite bag a filter assembly for depleting leukocytes from PRP; in the same way, PRC is leukocyte depleted by interposing between the blood collection bag and a second satellite bag a second filter assembly for removing leukocytes from PRC.

EP-A-0 879 680 describes a blood bag system for separating blood into blood components wherein leukocyte-free PRC is prepared by passing erythrocytes resuspended in an additive agent through a leukocyte filter.

The leukocyte filters, to which the present invention relates, typically comprise a housing with inlet and outlet ports and at least a porous element, within the housing, interposed between the inlet and outlet port. The said porous element usually consists of a web or mat which may be formed by one or more layers of filtering material, typically a non-woven fabric, which may or may not be bonded to each other.

According to the prior art, the porous elements can be produced from any material compatible with blood which is capable of forming fibers including natural or synthetic fibers. The preferred materials are synthetic polymers such as particularly polyolefines, polyesters, and polyamide; polybutylenterephthalate (PBT) is currently considered as a preferred polymer.

A parameter which is considered in the design of the porous elements is the critical surface tension (CST) of the employed material. The CST of a surface is a measure of the repellent properties of that surface; it is the maximum surface tension for a liquid that has a contact angle θ = 0°. CST cannot be measured directly on textile yarns that are complex bundles of twisted fibers; however, the CST for yarns may approximate that for chemically related plane surfaces.

With reference to the materials used in filters for leukocyte depletion, EP-A-0 313 348 describes an empirical method for measuring the critical surface tension of a porous medium, therein defined as "critical Wetting Surface Tension" (CWST). According to said method, the CWST of a porous medium is determined by individually applying to its surface, preferably drop wise, a series of liquids with a surface tension varying by 2 to 4 dyn/cm (mN/m) and observing the absorption and non-absorption of each liquid. The CWST of the porous medium in units of dyn/cm is defined as the mean value of the surface tension of the liquid which is absorbed and that of a liquid of neighboring surface tension which is not absorbed. Liquids with surface tension lower than the CWST of a porous medium or the CST of a given material will spontaneously wet the medium or material on contact. With reference to water (surface tension 72 dyn/cm), materials having a CST lower than the surface tension of water, will not be wetted. The CST of a material can therefore be taken as measure of the hydrophilicity of the material itself; the higher the CST or CWST, the higher is the hydrophylicity of the material.

EP-A-0 313 348 describes a device for the depletion of the leukocyte content of a blood product comprising a first porous element for removing gels, a second porous element for removing micro-aggregates and a third element for removing leukocytes, wherein at least the third element has been modified to a critical wetting surface tension in the range of from greater than 53 dyn/cm to less than 90 dyn/cm and wherein each successive element, from inlet to outlet, has a smaller pore diameter than that preceding it.

In these filters, gels and micro-aggregates are removed before filtering leukocytes. In the working examples of EP-A-0 313 348 the device comprises a first layer for gel filtration made of acrylic bonded needle punched PET, with a CWST of 50 and a plurality of additional layers for micro-aggregate removal and leukocyte adsorption having increased CWST which are typically made of melt-blown PBT which is surface grafted to increase its CWST. When the final porous element for leukocyte removal is made of a plurality of layers, these are usually made of the same material.

US-5,580,465 describes a method for preparing platelets by passing platelet-rich plasma (PRP) through a filter comprising a porous medium with a CWST of at least 70 dyn/cm under conditions sufficient to remove about 99.99% of the leukocytes from the PRP. From this document it can be derived that the increased hydrophylicity of the modified PBT filter element is enhancing the recovery of thrombocytes.

US-4,963,260 describes a liquid filtering device for separating leukocytes comprising a first filter element and a second filter element in a position downstream of the first one, wherein the second filter element is made of a material having a larger filtration resistance than the first filter element; filter resistance can be increased by decreasing pore size which also regulates the liquid pressure.

EP 0 542 655 discloses a filter device for separating agglutinated erythrocytes making use of a composite membrane comprising a highly hydrophilic first element (nylon 66), a second moderately hydrophobic intermediate element (polypropylene) and a third highly hydrophobic element (polytetrafluoroethylene) in the direction of filtration.

In view of the available prior art there is still a need for leukocyte filter devices having improved efficiency in leukocyte removal and better overall performance.

To this end, the present invention provides a filter device for the depletion of the leukocyte content from blood products according to claim 1.

Further characterizing features of the filter device according to the invention are defined in the appended claims.

As noted before, the CST of the filtering material or the CWST of a porous fabric made of said material can be taken as a measure of the hydrophylicity of the employed material, whereby a higher hydrophylicity corresponds to a higher CST or CWST.

Each porous element for leukocyte depletion may be comprised of one or more layers of adjacent filtering sheet material, which layers may optionally be bonded to each other. When a porous element is made of two or more layers, these will usually be made of the same filtering material having the same filtering and hydrophilicity properties. However, the said filtering layers may have different pore size, such as optionally a decreasing pore size from inlet to outlet, as suggested by the prior art.

The filter according to the invention may include within the filter housing upstream of the first porous element for leukocyte depletion, one or more porous elements specifically adapted for gel or micro-aggregate removal from the blood product.

The constructive principle underlying the invention is not intended to be limited to any specific filtering material and, in principle, any commercially available filtering material which is compatible with blood can be used. The filtering material is however preferably based on polyesters, such as PET or PBT or on more hydrophobic polymers such as polyolefines, particularly polypropylene, or polyamide.

The said hydrophobic polymer material can be rendered more hydrophilic by coating the fibers of the material with a more hydrophilic polymer such as particularly hydrophilic acrylic polymers or copolymers or hydrophilic polyurethane. The polymeric material can also be rendered more hydrophilic by surface grafting the polymeric material, particularly PBT, with compounds containing an ethylenically unsaturated group, such as an acrylic moiety combined with hydroxyl groups or methylacrylate or methylmethacrylate and combinations thereof, as described in EP-A-0 313 348. The layers of the filtering elements are usually made of a non-woven fabric obtained from fibers of the polymeric materials; however, also porous membranes or sintered porous media could be used in principle.

The absolute value of the CWST of the inlet filtering layer and successive layers can be selected in a wide range, according to the principles known in the art and depending upon the blood product (whole blood or other hemocomponents) which is to be leukocyte depleted by passing through the filter device. Preferably, the CWST of the inlet layer is of at least 53 dyn/cm (pure PBT) and more preferably of at least 63 dyn/cm.

The minimum and maximum wettability (CWST) difference between the first and last filter layer from inlet to outlet may vary in a wide range and may depend upon the distance between the bag containing the blood product and the layer, that is on the pressure generated by the column of liquid which is available for driving the blood product through the filter.

A preferred minimum wettability (CWST) difference is of about 10 dyn/cm, particularly when the inlet layer has a CWST of about 63 and independently from the distance between the bag and the filter device. A preferred maximum wettability (CWST) difference is of about 20 dyn/cm when the CWST of the outlet layer has a value of 53 dyn/cm (pure PBT).

As stated before, a preferred embodiment of the invention contemplates the use of a plurality of filter elements each consisting of a set of filter layers, such as particularly from 3 to 8 sets each comprising a plurality of layers, such as preferably from 2 to 50 layers. The difference in the wettability (CWST) of adjacent sets of layers can be in the range of from 2 to 50 dyn/cm.

The subject matter of the invention also includes a blood bag device including at least a primary and a satellite bag, connected by a flexible conduit wherein a filter device according to the invention is interposed in the flexible conduit for leukocyte depletion.

In a preferred embodiment of such a device the distance between the primary bag and the filter element is 20 to 80 cm, more preferably between 30 and 50 cm.

A further subject of the invention is the use of the described filter device for leukocyte depletion of blood products. The blood products include whole blood and other hemocomponents such as particularly platelet-rich plasma (PRP), packed red cells (PRC), platelet concentrate (PC) and plasma (PL).

In order to allow the user to properly set up the filter device of the invention in the blood bag system, the housing of the filter device may include appropriate indicia allowing to identify the inlet and outlet ports so that the filter device is mounted according to the appropriate negative hydrophilicity gradient from inlet to outlet.

It has been found that in the filter according to the invention the first set (or filter element) of more hydrophilic layers is easily wetted by the liquid and therefore the liquid pressure is distributed evenly on the following more hydrophobic layers. With this filter set-up regional pressure differences because of gas bubbles attached to the surface of the filter can be prevented, which decreases the risk of micro-ruptures in the filter.

The described arrangement improves the air elimination from the filter material, avoiding blood flow channeling, leading to a better leukocyte removal efficiency; moreover, by better exploiting the whole filtering material, its quantity can be reduced with consequent reduced cell loss.

A further advantage of a filtration system containing described filter element is that due to the improved wettability of the inlet layer the needed priming pressure as well as the priming time of the filter is reduced.

For the same reason the gravity pressure head which is needed for the efficient filtration of the liquid can be reduced. Therefore the distance needed between the primary bag and the filter element is smaller which is saving both space and tubing material.

In contrast to a filter with an inlet layer of 53 dyn/cm for which a distance to the primary bag of at least 80 cm is needed, for a filter with an inlet layer of 68 dyn/cm a distance of 30 cm is sufficient for efficient filtration of blood or blood components.

The following examples do not form part of the invention.

### Example 1

The filter device consists of 39 layers of PBT (50 g/m², CWST 53 dyn/cm) and one layer of polypropylene (10 g/m², CWST 33 dyn/cm). The filter device is used with a conventional blood bag system and is mounted in the flexible conduit connecting the primary bag to a first satellite bag at a distance of 80 cm from the primary bag. Whole blood is filtered through the filter device and the following features and parameters are obtained:
Filter priming time: 3 min.
Filtration time of one whole blood unit (about 450cm³): 28 min.
Blood volume recovery: 92%
Residual white blood cells: 200,000/unit

By way of comparison, a filter device was used without the polypropylene layer which was replaced by a PBT layer so as to achieve the same filter volume; the white blood cell contamination was found to be 900,000/unit.

### Example 2

Use is made of a filter consisting of 35 layers of coated PBT (50g/m²) wettable with a 68 dyn/cm liquid and 5 layers of uncoated PBT (50g/m²), CWST 53 dyn/cm; the filter was placed at the distance from the primary bag of 30 cm.
Filter priming time: 1,5 min.
Filtration time of one whole blood unit (about 450 cm³) : 20 min.
Blood volume recovery: 92%
Residual white blood cells: 100,000/unit

For a filter with a CWST of 53 dyn/cm without the hydrophilicity gradient the white blood cell contamination was found to be 1,100,000/unit.

### Example 3

Use is made of a filter consisting of 5 packages of 5 layers each of coated and uncoated PBT which were stacked together as follows:
First set: 63 dyn/cm
Second set: 61 dyn/cm
Third set: 59 dyn/cm
Fourth set: 55 dyn/cm
Fifth set: 52 dyn/cm
Distance bag/filter: 30 cm
Filter priming time: 1 min.
Filtration time of one whole blood unit (about 450 cm³) : 15 min.
Blood volume recovery: 92%
Residual white blood cells: 50,000/unit.

## Claims

1. A filter device for the depletion of the leukocyte content from blood products comprising a housing with an inlet port and an outlet port and, within said housing, interposed between the inlet port and the outlet port, more than two porous elements, each porous element comprising one or more layers of filtering material, **characterized in that** any given porous element is made of a filtering material having a pore size higher than the pore size of its successive porous element, in the direction of flow, from inlet port to outlet port, of the blood product through the filter device, and wherein said more than two porous elements have a different hydrophilicity and are arranged in the filter device so that any given porous element has a higher hydrophilicity than its successive porous element in the direction of flow, from inlet port to outlet port, of the blood product through the filter device.

2. A filter device according to claim 1, wherein each porous element comprises at least two adjacent layers of filtering material.

3. A filter device according to claim 2, wherein said at least two layers of filtering material are made of the same material having the same hydrophilicity properties.

4. A filter device according to claim 2 or 3, wherein said at least two layers have a decreasing pore size in the direction of flow, from inlet port to outlet port, of the blood product through the filter device.

5. A filter device according to any of claims 1 to 4, wherein said porous elements are made of fibers of a polymeric material selected from the group consisting of polyester, polyolefines, polyamide and polyester, polyolefines or polyamides coated with a hydrophilic polymer and mixtures of said fibers.

6. A filter device according to claim 5, wherein said hydrophilic polymer is selected from the group consisting of hydrophilic acrylic polymers or copolymers and hydrophilic polyurethane.

7. The filter device of claim 1, wherein a first porous element, in the direction of flow, from inlet to outlet, of the blood product through the filter device, is made of layers of polybutylenterephthalate fibers coated with a hydrophilic polymer or copolymer and wherein a second porous element, in the direction of flow, from inlet to outlet, of the blood product through the filter device, is made of uncoated polybutylenterephthalate or polypropylene layers.

8. A filter device according to any of the preceding claims wherein the difference between the hydrophilicity of the first porous element, in the direction of flow, from inlet port to outlet port, of the blood product through the filter device, and the final porous element, in the direction of flow, from inlet port to outlet port, of the blood product through the filter device, as measured by the value of the CST or CWST of the constituting material is of at least 10 dyn/cm.

9. A filter device according to any of the preceding claims, wherein the difference between the hydrophilicity of the first porous element, in the direction of flow, from inlet port to outlet port, of the blood product through the filter device, and the final porous element, in the direction of flow, from inlet port to outlet port, of the blood product through the filter device, as measured by the value of the CST or CWST of the constituting material is of from 10 to 20 dyn/cm.

10. A filter device according to any of the preceding claims wherein the first porous element, in the direction of flow, from inlet port to outlet port, of the blood product through the filter device, is made of material having a hydrophilicity as measured by the CST or CWST of the constituting material higher than 63 dyn/cm.

11. A filter device according to any of claims 1 to 6 comprising within said housing one or more additional filter elements of any hydrophilicity for removal of gel or micro-aggregate.

12. A filter device according to claim 11 wherein said filter elements for removal of gel or micro-aggregate are located upstream of the first porous elements for leukocyte removal, in the direction of flow, from inlet port to outlet port, of the blood product through the filter device.

13. A blood bag device for the separation of blood into leukocyte depleted blood components comprising at least a first bag connected, in fluid flow communication with a second bag through a leukocyte filter device according to any of claims 1 to 10.

14. A method for the leukocyte depletion of blood products comprising feeding said blood product through a filter device according to any of claims 1 to 11.

15. A method according to claim 14, wherein said blood product is selected from the group consisting of whole blood, platelet-rich plasma, packed red cells, platelet concentrate and plasma.

## Patentansprüche

1. Eine Filtervorrichtung zur Depletion des Leukozytengehaltes von Blutprodukten umfassend ein Gehäuse mit einem Einlassanschluss und einem Auslassanschluss und, innerhalb des Gehäuses, zwischen dem Einlassanschluss und dem Auslassanschluss gelegen, mehr als zwei poröse Elemente, jedes poröse Element umfassend einen oder mehrere Schichten von Filtermaterial, **gekennzeichnet dadurch, dass** jedes bestimmte poröse Element aus Filtermaterial mit einer Porengröße größer als die Porengröße des folgenden porösen Elementes in Richtung des Flusses des Blutproduktes durch die Filtervorrichtung vom Einlassanschluss zum Auslassanschluss hergestellt wird, und wobei die mehr als zwei porösen Elemente eine unterschiedliche Hydrophilie haben und in der Filtervorrichtung so angeordnet sind, dass jedes bestimmte poröse Element eine höhere Hydrophilie hat als das folgende poröse Element in Richtung des Flusses des Blutproduktes durch die Filtervorrichtung vom Einlassanschluss zum Auslassanschluss.

2. Eine Filtervorrichtung gemäß Anspruch 1, wobei jedes poröse Element mindestens zwei benachbarte Schichten Filtermaterial umfasst.

3. Eine Filtervorrichtung gemäß Anspruch 2, wobei die mindestens zwei Schichten Filtermaterial aus demselben Material mit denselben Hydrophilieeigenschaften hergestellt sind.

4. Eine Filtervorrichtung gemäß Anspruch 2 oder 3, wobei die mindestens zwei Schichten eine abnehmende Porengröße in Richtung des Flusses des Blutproduktes durch die Filtervorrichtung vom Einlassanschluss zum Auslassanschluss haben.

5. Eine Filtervorrichtung gemäß irgendeinem der Ansprüche 1 bis 4, wobei die porösen Elemente hergestellt sind aus Fasern eines polymeren Materials ausgewählt aus der Gruppe bestehend aus Polyester, Polyolefinen, Polyamid und mit einem hydrophilen Polymer beschichteten Polyestern, Polyolefinen oder Polyamiden und Mischungen der besagten Fasern.

6. Eine Filtervorrichtung gemäß Anspruch 5, wobei das hydrophile Polymer ausgewählt ist aus der Gruppe bestehend aus hydrophilen acrylischen Polymeren oder Kopolymeren und hydrophilem Polyurethan.

7. Die Filtervorrichtung aus Anspruch 1, wobei ein erstes poröses Element in Richtung des Flusses des Blutproduktes durch die Filtervorrichtung vom Einlassanschluss zum Auslassanschluss aus Schichten von mit einem hydrophilen Polymer oder Kopolymer beschichteten Polybutylenterephthalat-Fasern hergestellt ist und ein zweites poröses Element in Richtung des Flusses des Blutproduktes durch die Filtervorrichtung vom Einlassanschluss zum Auslassanschluss aus Schichten von unbeschichtetem Polybutylenterephthalat oder Polypropylen hergestellt ist.

8. Eine Filtervorrichtung gemäß irgendeinem der vorhergehenden Ansprüche, wobei der Unterschied zwischen der Hydrophilie des ersten porösen Elementes in Richtung des Flusses des Blutproduktes durch die Filtervorrichtung vom Einlassanschluss zum Auslassanschluss und des letzten porösen Elementes in Richtung des Flusses des Blutproduktes durch die Filtervorrichtung vom Einlassanschluss zum Auslassanschluss, gemessen über den Wert des CST oder CWST der sie bildenden Materialien, mindestens 10 dyn/cm ist.

9. Eine Filtervorrichtung gemäß irgendeinem der vorhergehenden Ansprüche, wobei der Unterschied zwischen der Hydrophilie des ersten porösen Elementes in Richtung des Flusses des Blutproduktes durch die Filtervorrichtung vom Einlassanschluss zum Auslassanschluss und des letzten porösen Elementes in Richtung des Flusses des Blutproduktes durch die Filtervorrichtung vom Einlassanschluss zum Auslassanschluss, gemessen über den Wert des CST oder CWST der sie bildenden Materialien, zwischen 10 bis 20 dyn/cm liegt.

10. Eine Filtervorrichtung gemäß irgendeinem der vorhergehenden Ansprüche, wobei das erste poröse Element in Richtung des Flusses des Blutproduktes durch die Filtervorrichtung vom Einlassanschluss zum Auslassanschluss aus Material mit einer Hydrophilie, gemessen über den Wert des CST oder CWST der es bildenden Materialien, von über 63 dyn/cm hergestellt ist.

11. Eine Filtervorrichtung gemäß irgendeinem der Ansprüche 1 bis 6 umfassend innerhalb des Gehäuses einen oder mehr zusätzliche Filterelemente einer beliebigen Hydrophilie zur Entfernung von Gel oder Mikroaggregat.

12. Eine Filtervorrichtung gemäß Anspruch 11, wobei die Filterelemente zur Entfernung von Gel oder Mikroaggregat flussaufwärts von dem ersten porösen Element zur Leukozytenentfernung in Richtung des Flusses des Blutproduktes durch die Filtervorrichtung vom Einlassanschluss zum Auslassanschluss liegen.

13. Eine Blutbeutelvorrichtung zur Trennung von Blut in leukozytendepletierte Blutkomponenten umfassend mindestens einen ersten Beutel verbunden in Flüssigkeitsfluss-Kommunikation mit einem zweiten Beutel durch einen Leukozytenfilter gemäß irgendeinem der Ansprüche 1 bis 10.

14. Ein Verfahren zur Leukozytendepletion von Blutprodukten umfassend Durchleiten des Blutproduktes durch eine Filtervorrichtung gemäß irgendeinem der Ansprüche 1 bis 11.

15. Ein Verfahren gemäß Anspruch 14, wobei das Blutprodukt ausgewählt ist aus der Gruppe bestehend aus Vollblut, plättchenreichem Plasma, gepackten roten Zellen, Plättchenkonzentrat und Plasma.

## Revendications

1. Dispositif de filtration pour la réduction de la teneur en leucocytes des produits sanguins, comprenant un logement comportant un orifice d'entrée et un orifice de sortie et, à l'intérieur dudit logement, interposés entre l'orifice d'entrée et l'orifice de sortie, plus de deux éléments poreux, chaque élément poreux comprenant une ou plusieurs couches d'un matériau filtrant,
**caractérisé en ce qu'**un quelconque élément poreux donné est constitué d'un matériau filtrant ayant un diamètre des pores supérieur au diamètre des pores de l'élément poreux qui le suit, dans le sens de l'écoulement, de l'orifice d'entrée vers l'orifice de sortie, du produit sanguin à travers le dispositif de filtration, et dans lequel lesdits plus de deux éléments poreux ont un caractère hydrophile différent et sont agencés dans le dispositif de filtration de telle manière qu'un quelconque élément poreux donné présente un caractère hydrophile supérieur à celui de l'élément poreux qui le suit dans le sens de l'écoulement, de l'orifice d'entrée vers l'orifice de sortie, du produit sanguin à travers le dispositif de filtration.

2. Dispositif de filtration selon la revendication 1, dans lequel chaque élément poreux comprend au moins deux couches adjacentes d'un matériau filtrant.

3. Dispositif de filtration selon la revendication 2, dans lequel lesdites au moins deux couches de matériau filtrant sont constituées du même matériau ayant les mêmes propriétés hydrophiles.

4. Dispositif de filtration selon la revendication 2 ou 3, dans lequel lesdites au moins deux couches ont un diamètre des pores qui diminue dans le sens de l'écoulement, de l'orifice d'entrée vers l'orifice de sortie, du produit sanguin à travers le dispositif de filtration.

5. Dispositif de filtration selon l'une quelconque des revendications 1 à 4, dans lequel lesdits éléments poreux sont constitués de fibres d'un matériau polymère choisi dans le groupe constitué par le polyester, les polyoléfines, le polyamide et le polyester, les polyoléfines ou les polyamides enduits d'un polymère hydrophile et les mélanges desdites fibres.

6. Dispositif de filtration selon la revendication 5, dans lequel ledit polymère hydrophile est choisi dans le groupe constitué par les polymères ou les copolymères acryliques hydrophiles et le polyuréthanne hydrophile.

7. Dispositif de filtration selon la revendication 1, dans lequel un premier élément poreux, dans le sens de l'écoulement, de l'entrée vers la sortie, du produit sanguin à travers le dispositif de filtration, est constitué de couches de fibres de téréphtalate de polybutylène enduites d'un polymère ou d'un copolymère hydrophile et dans lequel un second élément poreux, dans le sens de l'écoulement, de l'entrée vers la sortie, du produit sanguin à travers le dispositif de filtration, est constitué de couches de téréphtalate de polybutylène ou de polypropylène non enduites.

8. Dispositif de filtration selon l'une quelconque des revendications précédentes, dans lequel la différence entre le caractère hydrophile du premier élément poreux, dans le sens de l'écoulement, de l'orifice d'entrée vers l'orifice de sortie, du produit sanguin à travers le dispositif de filtration, et du dernier élément poreux, dans le sens de l'écoulement, de l'orifice d'entrée vers l'orifice de sortie, du produit sanguin à travers le dispositif de filtration, mesuré par la valeur de la tension superficielle critique (CST) ou de la tension superficielle critique de mouillage (CWST) du matériau constitutif est d'au moins 10 dyn/cm.

9. Dispositif de filtration selon l'une quelconque des revendications précédentes, dans lequel la différence entre le caractère hydrophile du premier élément poreux, dans le sens de l'écoulement, de l'orifice d'entrée vers l'orifice de sortie, du produit sanguin à travers le dispositif de filtration, et du dernier élément poreux, dans le sens de l'écoulement, de l'orifice d'entrée vers l'orifice de sortie, du produit sanguin à travers le dispositif de filtration, mesuré par la valeur de la tension superficielle critique (CST) ou de la tension superficielle critique de mouillage (CWST) du matériau constitutif va de 10 à 20 dyn/cm.

10. Dispositif de filtration selon l'une quelconque des revendications précédentes, dans lequel le premier élément poreux, dans le sens de l'écoulement, de l'orifice d'entrée vers l'orifice de sortie, du produit sanguin à travers le dispositif de filtration, est constitué d'un matériau ayant un caractère hydrophile, mesuré par la tension superficielle critique (CST) ou la tension superficielle critique de mouillage (CWST) du matériau constitutif, supérieur à 63 dyn/cm.

11. Dispositif de filtration selon l'une quelconque des revendications 1 à 6, comprenant à l'intérieur dudit logement un ou plusieurs éléments filtrants supplémentaires d'un caractère hydrophile quelconque pour l'élimination d'un gel ou d'un microagrégat.

12. Dispositif de filtration selon la revendication 11, dans lequel lesdits éléments filtrants pour l'élimination du gel ou du microagrégat se situent en amont du premier élément poreux pour l'élimination des leucocytes, dans le sens de l'écoulement, de l'orifice d'entrée vers l'orifice de sortie, du produit sanguin à travers le dispositif de filtration.

13. Dispositif à poches de sang pour la séparation du sang en composants sanguins à teneur en leucocytes réduite, comprenant au moins une première poche reliée, en communication d'écoulement fluidique, à une seconde poche par un dispositif de filtration des leucocytes selon l'une quelconque des revendications 1 à 10.

14. Procédé de réduction des leucocytes des produits sanguins, comprenant le passage dudit produit sanguin à travers un dispositif de filtration selon l'une quelconque des revendications 1 à 11.

15. Procédé selon la revendication 14, dans lequel ledit produit sanguin est choisi dans le groupe constitué par un sang total, un plasma riche en plaquettes, des globules rouges concentrés, un concentré plaquettaire et un plasma.
